# FASCICULE DE BREVET EUROPEEN

(11) **EP 0 614 362 B1**
(45) Date de publication et mention de la délivrance du brevet: **19.02.1997**
(21) Numéro de dépôt: 93901804.0
(22) Date de dépôt: 01.12.1992
(51) Int. Cl.: A61K 31/445, A61K 9/14, A61K 9/20

(54) **COMPOSITIONS PHARMACEUTIQUES A BASE D'EBASTINE OU DE SES ANALOGUES**
EBASTIN ODER EBASTIN-ANALOGA ENTHALTENDE ARZNEIMITTEL
PHARMACEUTICAL COMPOSITIONS BASED ON EBASTINE OR ANALOGUES THEREOF

(30) Priorité: 03.12.1991 FR 9114936
(43) Date de publication de la demande: 14.09.1994
(73) Titulaire: RHONE-POULENC RORER S.A., 92160 Antony (FR)
(72) Inventeur: BOBEE, Jean-Marc, F-91370 Verrières-le-Buisson (FR); CONRATH, Guillaume, F-92160 Antony (FR); GOUSSET, Gabriel, F-92350 Le Plessis-Robinson (FR); PONSOT, Michel, F-92260 Fontenay-aux-Roses (FR); VEILLARD, Michel, F-92330 Sceaux (FR)
(74) Mandataire: Savina, Jacques
(86) Numéro de dépôt international: FR9201116
(87) Numéro de publication internationale: WO9310782

(56) Documents cités:
- EP-A- 0 134 124
- WO-A-88/08302
- WO-A-88/09656
- WO-A-89/10143

## Description

La présente invention concerne une nouvelle forme pharmaceutique à base de dérivés de la piperidine substitués en 1,4.

Les composés pharmaceutiques objet de la formulation décrite dans la présente invention sont décrits dans la demande de brevet européen EP 134 124 incluse dans la présente demande par référence.

Ces composés et encore plus spécifiquement le composé suivant dont la dénomination internationale est l'ébastine ou
4-diphénylméthoxy 1-[3-(4-ter-butylbenzoyl)propyl] piperidine de formule: 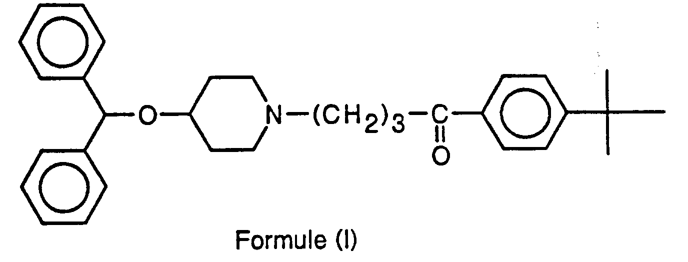 présentent, lorsqu'ils sont formulés sous forme solide, une biodisponibilité très médiocre. Cette biodisponibilité insuffisante est en partie liée à une mauvaise dissolution dans l'eau. Il est ainsi difficile à partir de la matière première brute, décrite dans le brevet préalablement mentionné, de la mettre sous une forme solide utilisable par l'homme, et présentant une biodisponibilité et par conséquent une activité biologique correcte. Le composé de formule (I), sous forme de sel et plus particulièrement sous forme de lactate présente un optimum de solubilité à pH 2, qui est égal à 0,8 mg/ml, le composé de formule (I) sous forme basique non salifié présente une solubilité dans l'eau encore plus faible.

Ces composés ont une activité antihistaminique H1 et sont utiles dans le traitement des maladies respiratoires, allergiques ou cardiovasculaires. Ils relâchent ainsi in vitro et in vivo les muscles lisses vasculaires et bronchiques.

Ils inhibent aussi l'effet constricteur de l'adrénaline, des ions potassium tant au niveau intestinal qu'au niveau de la trachée. Ils bloquent ainsi la bronchoconstriction provoquée par les aérosols d'histamine à des doses aussi faibles que 1 mg/kg.

Ces composés sont actifs par voie parentérale ainsi que par voie orale. Lors de leur administration par voie orale le principe actif de formule (I), à cause de sa faible solubilité dans l'eau, demande un temps de dissolution très long dans le milieu aqueux de l'estomac, ce qui peut entrainer une perte de principe actif, non dissous et donc non absorbable, lors de la vidange gastrique et donc entrainer l'utilisation d'un surdosage toujours dangeureux dans le domaine médical. Ainsi dans la demande de brevet préalablement citée c'est à dire la demande de brevet européen EP 134 124, lorsque l'on prépare des comprimés, selon l'exemple 9 qui est le seul à décrire une forme comprimé, la dissolution du principe actif dans un milieu acide est extrêmement lente.

La présente invention a cherché à mettre au point une nouvelle forme solide d'administration des composés de formule (II), ci-dessous, dans laquelle le principe actif présente une solubilité et donc une biodisponibilité améliorée.

La présente invention concerne une nouvelle forme solide à base de composés répondant à la formule (II) suivante: 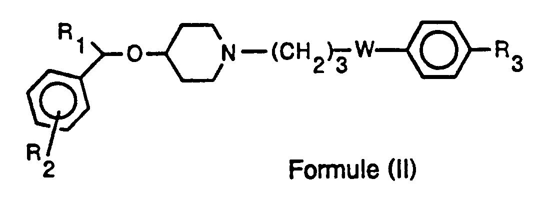 dans laquelle :
- R1 représente un groupe thiényl, un groupe phényl éventuellement substitué par un halogène, un groupe alcoxy contenant 1 à 6 atomes de carbone, un groupe alkyle contenant 1 à 6 atomes de carbone,
- R2 représente un atome d'halogène, un atome d'hydrogène, un groupe alcoxy contenant 1 à 6 atomes de carbone, un groupe alkyle contenant 1 à 6 atomes de carbone,
- R3 représente un atome d'halogène, un atome d'hydrogène, un groupe alcoxy contenant 1 à 6 atomes de carbone, un groupe alkyle contenant 1 à 6 atomes de carbone, un groupe alkylthio contenant 1 à 6 atomes de carbone, un groupe cycloalkyle contenant 5 ou 6 atomes de carbone ou un groupe de formule : 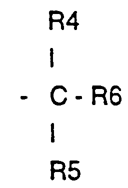 où R4 et R5 représentent indépendamment l'un de l'autre un atome d'hydrogène ou un groupe alkyle contenant 1 à 6 atomes de carbone, R6 représente un groupe cycloalkyle contenant 3 à 6 atomes de carbone, hydroxyméthyl, carboxy ou alcoxycarbonyle contenant 2 à 7 atomes de carbone,
- W représente un groupe carbonyle ou hydroxyméthylène, et leurs sels; caractérisée en ce que le principe actif de formule (II) est micronisée.

Cette forme présente l'avantage de présenter une vitesse de dissolution initiale d'environ 40 % supérieure à la forme pharamaceutique obtenue à partir d'un composé de formule (II) non micronisé.

Selon une forme encore améliorée de dissolution des composés de formule (II) on effectue en plus de la micronisation une hydrophilisation du produit, ce qui lui permet d'atteindre une vitesse de dissolution améliorée d'environ 300 % par rapport à la forme solide comportant un composé de formule (II) n'ayant subi ni étape de micronisation, ni étape d'hydrophilisation.

Selon un exemple de mise en oeuvre de l'invention, on introduit dans un microniseur muni d'une manche filtrante et d'une trémie d'alimentation le composé de formule (I) brut cristallisé. On injecte de l'air comprimé et refroidi de façon à fractionner les particules. Le produit micronisé obtenu qui est préféré présente les caractéristiques suivantes:
- taille maximale inférieure à 200 µm
- granulométrie moyenne en nombre comprise entre 0,5 et 15 µm
- avec de préférence 90 %, en nombre,de particules ayant une granulométrie inférieure à 25 µm et de préférence inférieure à 20 µm.

La poudre micronisée peut ensuite être mise en forme pharmaceutique finale par exemple sous forme comprimée par compression directe ou granulation humide, ou encore sous forme de lyophilisats oraux ou de gélules.

Selon une seconde meilleure manière de mettre en oeuvre l'invention on hydrophilise la poudre micronisée obtenue. L'hydrophilisation est réalisée par pulvérisation d'eau. La quantité d'eau introduite est comprise de préférence entre 10 et 30 g d'eau pour 100 g de poudre micronisée. Cette hydrophilisation peut être réalisée en même temps que la granulation, on effectue alors une granulation humide.

Pour la mise en forme comprimée on peut soit réaliser une compression directe sur la poudre micronisée et non hydrophilisée, soit réaliser une compression sur la poudre hydrophilisée et granulée.

Pour la mise sous forme de lyophilisats oraux on mélange le prinipe actif de formule (II) avec:
- un agent édulcorant choisi par exemple parmi l'aspartam ou le saccharinate de sodium
- un agent liant tel que notamment le dextran
- un agent diluant tel que par exemple le mannitol ou le lactose.

La présente invention sera plus complètement décrite à l'aide des exemples suivants qui ne doivent pas être considérés comme limitatifs de l'invention.

### MICRONISATION

Elle est réalisée dans un microniseur de diamètre 200 mm en acier inoxydable ayant une manche filtrante en polyester. La trémie d'alimentation est aussi en acier inoxydable. L'air comprimé utilisé est deshydraté par réfrigération.

L'injection d'air comprimé provoque l'accélération des particules dans l'assiette du microniseur ; les particules qui entrent en collision entre elles se fractionnent en particules de plus en plus fines.

| PRODUIT BRUT | PRODUIT MICRONISE |
|---|---|
| 97,7 % < 500 µ | 100 % < 80 µ |
| 94,4 % < 400 µ | 99,4 % < 50 µ |
| | 97,6 % < 25 µ |
| 76 % < 225 µ | 96,6 % < 20 µ |
| 43,4 % < 125 µ | 93,1 % < 15 µ |
| 23,7 % < 83 µ | 90,3 % < 10 µ |
| 8,1 % < 46 µ | 61,1 % < 5 µ |
| 2,5 % < 20 µ | 31,1 % < 2 µ |
| | 18 % < 1 µ |
| Taille moyenne = 140 µm | Taille moyenne = 3,4 µ |

### PREPARATION DES COMPRIMES

Pour la préparation de cette composition on met en oeuvre les produits suivants :

| | |
|---|---|
| - Ebastine (composé de formule (I)) | 10.0 mg |
| - Cellulose microcristalline | 20.0 mg |
| - Lactose monohydraté | 88.5 mg |
| - Amidon prégélifié | 5.2 mg |
| - Carboxyméthylcellulose sodique réticulée | 5.0 mg |
| - Stéarate de Magnésium | 1.3 mg |
| Pour un comprimé de | 130.0 mg |

### EXEMPLE 1 ET COMPARATIF 1

### 1 - COMPRESSION DIRECTE - Mode opératoire (taille essai 520 g)

1° - Tamiser sur un tamis de 0.630 mm d'ouverture de maille les produits suivants :
   - Ebastine micronisée ou non micronisée (exemples 1 et C1)
   - Cellulose microcristalline de taille moyenne de particules égales à 200 µ
   - Lactose monohydraté de taille moyenne de particules égale à 200 µ
   - Amidon prégélifié
2° - Mélanger pendant 15 minutes dans un appareil type Turbula
3° - Tamiser le stéarate de magnésium et la carboxyméthylcellulose sodique réticulée sur un tamis de 0.5 mm d'ouverture de maille.
4° - Mélanger pendant 5 minutes dans un appareil type Turbula

### EXEMPLE 2 ET COMPARATIF 2

### 2 - GRANULATION HUMIDE - Mode opératoire (taille essai 520 g)

1° - Placer dans la cuve d'un petit mélangeur planétaire (type Kenwood) :
   - Cellulose microcristalline
   - Lactose monohydraté
   - Amidon prégélifié
   - Ebastine micronisée ou non micronisée (exemple 2 et C2)
2° - Mélanger pendant 10 minutes
3° - Mouiller avec 23 % d'eau purifiée (calcul fait par rapport à la masse totale mise en oeuvre).
4° - Granuler pendant 10 minutes.
5° - Sécher en étuve ventilée pendant 6.5 heures à 50°C.
6° - Egaliser le grain obtenu par passage sur un tamis de 0.8 mm d'ouverture de malle
7° - Tamiser le stéarate de magnésium et la carboxyméthylcellulose sodique réticulée sur un tamis de 0.5 mm d'ouverture de maille et ajouter ces deux excipients au grain précédemment obtenu.
8° - Mélanger à l'aide d'un appareil type Turbula pendant 10 minutes.

### 3 - COMPRESSION

Dans les deux cas, comprimer sur une machine alternative FROGERAIS OA équipée de poinçons de diamètre 7 mm et présentant un rayon de courbure de 8 mm.

Le poids moyen visé pour ces comprimés est de 130 mg. La machine est aussi réglée pour que ces comprimés présentent une résistance à la rupture de 50 Newtons (appareil Sleuninger).

On mesure le pourcentage de composé de formule (I) dissous, après 45 minutes, dans un milieu acide chlorhydrique 0,1 N. L'essai de dissolution est réalisé dans l'appareil à palette décrit à la pharmacopée Européenne 2ème édition V5.4 (1986) et à l'USP XXII 〈711〉. La prise d'essai est de 6 comprimés. Le milieu de dissolution contient 1000 ml d'acide chlorhydrique 0,1 N et la vitesse de rotation de la palette est de 100 tours/minute. Le dosage est effectué par spectrophotométrie dans l'ultra-violet à une longueur de 258 nanomètres en cuves de 1 cm (E 1 % 1 cm = 366). Les résultats sont indiqués sur le tableau (I)

| ESSAIS | COMPOSE DE FORMULE (I) | FABRICATION DU COMPRIME | TAUX DE DISSOLUTION |
|---|---|---|---|
| C1 | non micronisé | compression directe | 26,4 % |
| C2 | non micronisé | granulation humide | 36,7 % |
| 1 | micronisé | compression directe | 52,6 % |
| 2 | micronisé | granulation humide | 94,0 % |

### EXEMPLE 3 PREPARATION DE LYOPHILISATS ORAUX

On met en oeuvre la composition suivante:

| | |
|---|---|
| - Ebastine (micronisée) | 5,00 mg |
| - Gomme xanthane | 0,12 mg |
| - Aspartam | 5,00 mg |
| - Docusate de sodium | 0,25 mg |
| - Dextran 70 | 20,00 mg |
| - Mannitol | 669,63 mg |
| - eau | 525,00 mg |

On dissout le docusate de sodium dans 90 % de l'eau, sous agitation dans une turbine, 30 minutes, à température ambiante. On ajoute la gomme xanthane, on agite pendant 30 minutes jusqu'à dissolution complète. On disperse dans cette solution le dextran jusqu'à l'obtention d'une solution limpide.

Dans un mélangeur planétaire on introduit l'ébastine micronisée, l'aspartam et le mannitol, on mélange sous pression réduite (0,2 bar) pendant 30 minutes, puis on introduit la solution dans le mélange de poudres, on rince le containeur de la solution avec le reste de l'eau et on l'introduit dans le mélangeur. On maintient la pâte sous agitation pendant 30 minutes. On divise la pâte dans des alvéoles en PVC de 1,6 ml.

On réalise une cryodéssication dans un lyophilisateur.

On obtient des lyophilisats oraux d'une masse moyenne de 700 mg ayant un titre moyen en ébastine de 4,90 mg par lyophilisat et contenant 0,26 % d'eau.

Le goût des lyophilisats est frais et sucré sans amertume.

Le test de désagrégation à la cuillère est pratiqué et donne un temps moyen de 45 s.

Ce temps correspond à la durée nécessaire pour obtenir une désagrégation complète d'un lyophilisat oral soumis à une agitation constante (cuillère tournant à 200 tours/minute) dans un bécher de 400 ml contenant 200 ml d'eau distillée (température de l'eau = 20°C)

## Revendications

1. Composition pharmaceutique solide, à dissolution améliorée, contenant un composé répondant à la formule : 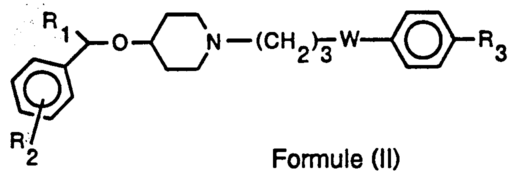 dans laquelle :
- R1 représente un groupe thiényl, un groupe phényl éventuellement substitué par un halogène, un groupe alcoxy contenant 1 à 6 atomes de carbone, un groupe alkyle contenant 1 à 6 atomes de carbone,
- R2 représente un atome d'halogène, un atome d'hydrogène, un groupe alcoxy contenant 1 à 6 atomes de carbone, un groupe alkyle contenant 1 à 6 atomes de carbone,
- R3 représente un atome d'halogène, un atome d'hydrogène, un groupe alcoxy contenant 1 à 6 atomes de carbone, un groupe alkyle contenant 1 à 6 atomes de carbone, un groupe alkylthio contenant 1 à 6 atomes de carbone, un groupe cycloalkyle contenant 5 ou 6 atomes de carbone ou un groupe de formule : 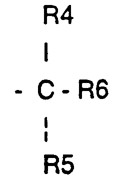 où R4 et R5 représentent indépendamment l'un de l'autre un atome d'hydrogène ou un groupe alkyle contenant 1 à 6 atomes de carbone, R6 représente un groupe cycloalkyle contenant 3 à 6 atomes de carbone, hydroxyméthyl, carboxy ou alcoxycarbonyle contenant 2 à 7 atomes de carbone,
- W représente un groupe carbonyle ou hydroxyméthylène, et leurs sels; caractérisée en ce que le composé de formule (II) est micronisé.

2. Composition selon la revendication 1 caractérisée en ce que le principe actif répond à la formule (II) dans laquelle:
- R1 représente un groupe phényle
- R2 représente l'hydrogène
- R3 représente un groupe tertiobutyle
- W représente un groupe carbonyl.

3. Composition selon les revendications 1 et 2 caractérisée en ce que le principe actif de formule (II) est hydrophilisé.

4. Composition selon la revendication 1 caractérisée en ce que le principe actif de formule (II) présente les caractéristiques suivantes:
- taille maximale inférieure à 200 µm
- granulométrie moyenne en nombre comprise entre 0,5 et 15 µm
- avec de préférence 90 %, en nombre,de particules ayant une granulométrie inférieure à 25 µm et de préférence inférieure à 20 µm.

5. Composition sous forme comprimée caractérisée en ce qu'elle contient le principe actif de la revendication 4.

6. Procédé de préparation de compositions selon la revendication 5 caractérisé en ce que:
- dans une première étape on réalise une micronisation du composé de formule (II)
- dans une deuxième étape on réalise une compression directe.

7. Procédé de préparation de compositions selon la revendication 5 caractérisé en ce que:
- dans une première étape on réalise une micronisation du composé de formule (II)
- dans une deuxième étape on réalise une granulation humide puis une compression.

8. Compositions sous forme de lyophilisats oraux caractérisées en ce quelles contiennent le principe actif selon la revendication 4, un agent suspensif choisi parmi les gommes, un agent édulcorant et un diluant.

## Claims

1. Solid pharmaceutical composition having improved dissolution properties, containing a compound corresponding to the formula: 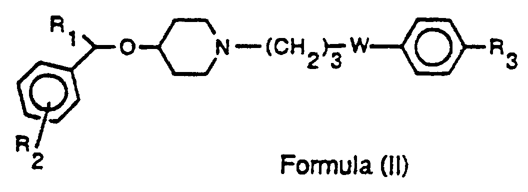 in which:
- R₁ represents a thienyl group, a phenyl group optionally substituted with a halogen, an alkoxy group containing 1 to 6 carbon atoms or an alkyl group containing 1 to 6 carbon atoms,
- R₂ represents a halogen atom, a hydrogen atom, an alkoxy group containing 1 to 6 carbon atoms or an alkyl group containing 1 to 6 carbon atoms,
- R₃ represents a halogen atom, a hydrogen atom, an alkoxy group containing 1 to 6 carbon atoms, an alkyl group containing 1 to 6 carbon atoms, an alkylthio group containing 1 to 6 carbon atoms, a cycloalkyl group containing 5 or 6 carbon atoms or a group of formula: 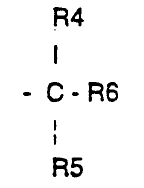 where R4 and R5 represent, independently of each other, a hydrogen atom or an alkyl group containing 1 to 6 carbon atoms, R6 represents a cycloalkyl group containing 3 to 6 carbon atoms or a hydroxymethyl or carboxyl group or an alkoxycarbonyl group containing 2 to 7 carbon atoms,
- W represents a carbonyl or hydroxymethylene group, and their salts; characterized in that the compound of formula (II) is micronized.

2. Composition according to claim 1, characterized in that the active principle corresponds to the formula (II) in which:
- R₁ represents a phenyl group
- R₂ represents hydrogen
- R₃ represents a tert-butyl group
- W represents a carbonyl group.

3. Composition according to claims 1 and 2, characterized in that the active principle of formula (II) is hydrophilized.

4. Composition according to claim 1, characterized in that the active principle of formula (II) has the following characteristics:
- maximum size smaller than 200 µm
- number-average particle size between 0.5 and 15 µm
- preferably with 90 %, by number, of particles having a particle size smaller than 25 µm, and preferably smaller than 20 µm.

5. Composition in compressed form, characterized in that it contains the active principle of claim 4.

6. Process for the preparation of compositions according to claim 5, characterized in that:
- a micronization of the compound of formula (II) is performed in a first step
- a direct compression is performed in a second step.

7. Process for the preparation of compositions according to claim 5, characterized in that:
- a micronization of the compound of formula (II) is performed in a first step
- a wet granulation, followed by a compression, is performed in a second step.

8. Compositions in the form of oral lyophilizates, characterized in that they contain the active principle according to claim 4, a suspension agent chosen from gums, a sweetener and a diluent.

## Patentansprüche

1. Feste pharmazeutische Zusammensetzung mit verbesserter Auflösung, enthaltend eine Verbindung der Formel 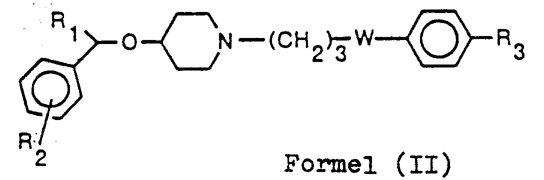 worin
- R₁ eine Thienylgruppe, eine gegebenenfalls durch ein Halogen substituierte Phenylgruppe, eine Alkoxygruppe mit 1 bis 6 Kohlenstoffatomen, eine Alkylgruppe mit 1 bis 6 Kohlenstoffatomen bedeutet,
- R₂ ein Halogenatom, ein Wasserstoffatom, eine Alkoxygruppe mit 1 bis 6 Kohlenstoffatomen, eine Alkylgruppe mit 1 bis 6 Kohlenstoffatomen darstellt,
- R₃ ein Halogenatom, ein Wasserstoffatom, eine Alkoxygruppe mit 1 bis 6 Kohlenstoffatomen, eine Alkylgruppe mit 1 bis 6 Kohlenstoffatomen, eine Alkylthiogruppe mit 1 bis 6 Kohlenstoffatomen, eine Cycloalkylgruppe mit 5 oder 6 Kohlenstoffatomen oder eine Gruppe der Formel 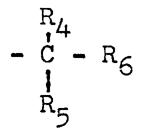 bedeutet, wobei R₄ und R₅ unabhängig voneinander ein Wasserstoffatom oder eine Alkylgruppe mit 1 bis 6 Kohlenstoffatomen darstellen, R₆ eine Cycloalkylgruppe mit 3 bis 6 Kohlenstoffatomen, Hydroxymethyl, Carboxy oder Alkoxycarbonyl mit 2 bis 7 Kohlenstoffatomen bedeutet,
- W für eine Carbonyl- oder Hydroxymethylengruppe steht und deren Salze,
dadurch gekennzeichnet, daß die Verbindung der Formel (II) mikronisiert ist.

2. Zusammensetzung gemäß Anspruch 1, dadurch gekennzeichnet, daß der Wirkstoff der Formel (II) entspricht, worin
- R₁ eine Phenylgruppe bedeutet
- R₂ Wasserstoff bedeutet
- R₃ eine tert.-Butylgruppe bedeutet
- W eine Carbonylgruppe bedeutet.

3. Zusammensetzung gemäß den Ansprüchen 1 und 2, dadurch gekennzeichnet, daß der Wirkstoff der Formel (II) hydrophilisiert ist.

4. Zusammensetzung gemäß Anspruch 1, dadurch gekennzeichnet, daß der Wirkstoff der Formel (II) die folgenden Eigenschaften aufweist:
- maximale Größe geringer als 200 µm
- zahlenmittlere Granulometrie zwischen 0,5 und 15 µm
- wobei bevorzugt 90%, bezogen auf Zahl, der Teilchen eine Granulometrie von geringer als 25 µm und bevorzugt geringer als 20 µm besitzen.

5. Zusammensetzung in komprimierter Form, dadurch gekennzeichnet, daß sie den Wirkstoff gemäß Anspruch 4 enthält.

6. Verfahren zur Herstellung von Zusammensetzungen gemäß Anspruch 5, dadurch gekennzeichnet, daß man
- in einer ersten Stufe eine Mikronisierung der Verbindung der Formel (II) vornimmt,
- in einer zweiten Stufe eine direkte Komprimierung vornimmt.

7. Verfahren zur Herstellung der Zusammensetzung gemäß Anspruch 5, dadurch gekennzeichnet, daß man
- in einer ersten Stufe eine Mikronisierung der Verbindung der Formel (II) vornimmt,
- in einer zweiten Stufe eine Feuchtgranulierung und anschließend eine Komprimierung vornimmt.

8. Zusammensetzungen in Form oraler Lyophilisate, dadurch gekennzeichnet, daß sie den Wirkstoff gemäß Anspruch 4, ein Suspendier-Mittel, ausgewählt unter den Gummen, ein süßendes Mittel und ein Verdünnungsmittel enthalten.
